# FASCICULE DE BREVET EUROPEEN

(11) **EP 0 717 981 B1**
(45) Date de publication et mention de la délivrance du brevet: **09.02.2000**
(21) Numéro de dépôt: 95402889.0
(22) Date de dépôt: 20.12.1995
(51) Int. Cl.: A61K 7/06

(54) **Composition cosmétique, dermatologique ou pharmaceutique stable contenant du disulfure de sélénium et au moins un sel de zinc**
Stabile kosmetische, dermatologische oder pharmazeutische Zusammensetzung, die Seleniumdisulfid und mindestens ein Zinksalz enthält
Stable cosmetic, dermatologic or pharmaceutic composition containing selenium disulfide and at least a zinc salt

(30) Priorité: 20.12.1994 FR 9415326
(43) Date de publication de la demande: 26.06.1996
(73) Titulaire: L'OREAL, 75008 Paris (FR)
(72) Inventeur: Cauwet, Danièle, F-75011 Paris (FR); Sebag, Henri, F-75016 Paris (FR)
(74) Mandataire: Stalla-Bourdillon, Bernard

(56) Documents cités:
- EP-A- 0 326 272
- FR-A- 2 587 208
- US-A- 2 694 669
- US-A- 4 854 333

## Description

La présente invention a pour objet une composition cosmétique, dermatologique ou pharmaceutique, stable, contenant du disulfure de sélénium et au moins un sel de zinc d'un acide minéral ou organo-carboxylique en tant qu'agent stabilisant. Plus particulièrement, la présente invention a pour objet une composition capillaire anti-pelliculaire.

En vue de combattre la formation des pellicules qui est généralement accompagnée d'une prolifération microbienne et/ou fongique, il a été proposé comme produits anti-pelliculaires soit des produits inhibant la prolifération microbienne, soit des produits kératolytiques. Parmi ces derniers, l'emploi du disulfure de sélénium a été tout particulièrement préconisé en raison de sa puissante activité cytostatique (voir Ch. Zviak "Science des traitements capillaires", Ed. 1988).

Il a ainsi été proposé dans EP-0.524.859 l'utilisation, dans des shampooings, du disulfure de sélénium en association avec un agent tensioactif non ionique du type alkylpolyglucoside ou polyglycérolé et en présence d'un agent de suspension choisi parmi les biopolysaccharides ou les celluloses anioniques.

Par ailleurs, il a été décrit dans EP-0.422.508 l'emploi, dans des shampooings, du disulfure de sélénium en association avec un sel de 2-mercaptopyridine N-oxyde ou de 1-hydroxy-2-pyrrolidone et en présence d'un agent tensioactif non ionique du type alkylpolyglucoside.

Cependant, si le disulfure de sélénium possède une excellente activité anti-pelliculaire, il présente néanmoins l'inconvénient de brunir progressivement dans le temps, passant de l'orangé au brun vert.

Afin de remédier à ce problème de changement de couleur, il a été proposé dans US-4.854.333, des compositions contenant en association avec le disulfure de sélénium, un agent oxydant du type peroxyde ou persel. Toutefois, l'emploi d'agents oxydants peut présenter des problèmes de toxicité et/ou de compatibilité dans les compositions.

Dans le brevet US-2 694 669, il a été décrit la stabilisation de compositions à base de "sesols", ces derniers étant des associations très particulières constituées, à part égale, de disulfure de sélénium et de bentonite.

Selon ce brevet, la stabilisation est obtenue à l'aide d'acides minéraux ou organiques ou de leurs sels notamment ceux de sodium et en particulier du monophosphate de sodium. Il est tout particulièrement recommandé selon ce brevet, d'utiliser en vue de stabiliser les "sesols", sur une longue période de temps, l'association d'un acide, d'un sel d'acide et d'un agent mouillant.

On a maintenant constaté de façon surprenante et inattendue qu'il était possible d'obtenir des compositions de couleur stable à base de disulfure de sélénium en utilisant en association un sel de zinc d'un acide minéral ou organe-carboxylique.

On entend par composition de couleur stable selon l'invention, une composition qui, conservée pendant au moins 1 mois à l'étuve à 45°C, ne présente pas de modification sensible de couleur par rapport à sa couleur initiale.

La présente invention a donc pour objet une composition cosmétique, dermatologique ou pharmaceutique à action anti-pelliculaire étant stable et contenant en milieu aqueux du disulfure de sélénium en mélange avec au moins un sel de zinc d'un acide minéral ou organo-carboxylique ayant de préférence de 2 à 6 atomes de carbone.

Parmi les sels de zinc d'un acide minéral, on peut citer en particulier le chlorure et le sulfate, mais de préférence le chlorure de zinc.

Parmi les sels de zinc d'un acide organo-carboxylique ayant de 2 à 6 atomes de carbone, on peut citer en particulier l'acétate, le glycolate, le lactate, le gluconate et le citrate, mais de préférence le lactate et le citrate de zinc.

Selon un mode de réalisation particulièrement préféré, les sels de zinc sont choisis parmi ceux solubles dans l'eau.

Le disulfure de sélénium utilisé dans les compositions selon l'invention comporte essentiellement un atome de sélénium pour deux atomes de soufre. Il peut avoir également une structure de polysulfure SeₓS_{y} dans laquelle x + y = 8.

Le disulfure de sélénium se présente sous forme d'une poudre dont les particules ont une granulométrie inférieure à 200 µm et de préférence inférieure à 25 µm.

Dans les compositions selon l'invention, le disulfure de sélénium est de préférence présent en une proportion comprise entre 0,001 % et 5 % en poids, et de préférence entre 0,25 % et 2 % en poids par rapport au poids total de la composition.

Le sel de zinc est généralement présent en une proportion comprise entre 0,01 % et 5% en poids, et de préférence entre 0,1 % et 3 % en poids par rapport au poids total de la composition.

Selon un mode de réalisation préféré des compositions selon l'invention, le rapport en poids entre le sel de zinc et le disulfure de sélénium est compris entre 0,1 et 3, et de préférence entre 0,2 et 2.

Les compositions selon l'invention peuvent se présenter sous différentes formes. Parmi celles-ci, on peut citer notamment les shampooings, les compositions à appliquer avant ou après un shampooing, celles-ci se présentant sous forme d'une lotion plus ou moins épaissie, d'un gel ou d'une émulsion.

Les compositions selon l'invention, notamment sous forme de shampooing, contiennent en outre au moins un agent tensioactif anionique, non-ionique, zwittérionique, amphotère ou cationique.

La proportion en agent tensioactif est généralement comprise entre 0,01 % et 50% en poids, mais de préférence entre 0,05 % et 30 % en poids par rapport au poids total de la composition.

Lorsque l'on utilise un agent tensioactif du type non-ionique, celui-ci est généralement utilisé en une proportion comprise entre 0,1 % et 40 % en poids, et de préférence entre 1 % et 20 % en poids par rapport au poids total de la composition.

Les agents tensioactifs du type cationique, en raison de leur faible pouvoir détergent, sont plus particulièrement utilisés dans les compositions selon l'invention sous forme de compositions de soins avant ou après shampooing.

Parmi les tensioactifs anioniques utilisables, seuls ou en mélanges, selon la présente invention, on peut citer notamment les sels alcalins, notamment de sodium, les sels d'ammonium, les sels d'amines, les sels d'aminoalcools ou les sels de magnésium, des composés suivants : les alkylsulfates, les alkyléthersulfates, les alkylamidoéthersulfates, les monoglycérides sulfates, les alkylglycérylsulfonates, les alkylsulfonates, les alkylphosphates, les alkylamide sulfonates, les alkylarylsulfonates, les α-oléfinesulfonates, les paraffines sulfonates, les alkylsulfosuccinates, les alkyléthersulfosuccinates, les alkylamidesulfosuccinates, les alkylsulfosuccinamates, les alkylsulfoacétates, les alkylétherphosphates, les acyliséthionates, les N-acyltaurates, les N-acylaminoacides tels que les N-acylsarcosinates, les N-acylglutamates.

Comme tensioactifs anioniques, on peut également utiliser des sels d'acides gras tels que ceux des acides undécénylique, oléique, ricinoléique, palmitique et stéarique, des acides d'huile de coprah ou d'huile de coprah hydrogénée ; des acylhydroxyacides tels que les acyl-lactylates.

On peut également utiliser des tensioactifs faiblement anioniques, comme les acides d'alkyl D-galactoside uroniques et leurs sels ainsi que les acides éthers carboxyliques polyoxyalkylénés ou leurs sels. Le radical alkyle ou acyle des différents tensio-actifs ci-dessus énumérés ayant de préférence de 8 à 22 atomes de carbone.

Parmi les tensioactifs non ioniques, on peut citer les alcools, les α-diols, les alkylphénols ou les acides gras polyéthoxylés, polypropoxylés ou polyglycérolés, ayant une chaîne grasse comportant de 8 à 22 atomes de carbone, le nombre de groupements d'oxyde d'éthylène ou de propylène pouvant aller de 2 à 50 et celui de glycérol notamment de 2 à 30. On peut également citer les copolymères d'oxyde d'éthylène et de propylène, les amines ou les amides gras polyéthoxylés ayant de préférence de 2 à 30 moles d'oxyde d'éthylène, les amides gras polyglycérolés comportant en moyenne 1 à 5 groupements glycéro, les diglycolamides polyglycérolés, les esters d'acides gras du sorbitan éventuellement oxyéthylénés, les esters d'acides gras du saccharose, les alkylpolyglycosides éventuellement oxyalkylénés, les esters d'alkylglucosides, les dérivés de N-alkylglucamine et les oxydes d'amine.

Parmi les tensioactifs amphotères ou zwittérioniques, on peut citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) tels que par exemple les alkylbétaines, les alkylaminocarboxylates, les sulfobétaines, les alkylamidoalkylbétaines, les alkylamidoalkylsulfobétaines, les dérivés d'imidazolium notamment ceux d'amphocarboxyglycinate ou d'amphocarboxypropionate.

Parmi les tensioactifs cationiques, on peut citer notamment les sels d'amines grasses éventuellement polyoxyalkylénées et/ou quaternisées, les esters d'acides gras et d'aminoalcools éventuellement polyoxyalkylénés et/ou quaternisés, les sels d'ammonium quaternaires tels que les chlorures ou les bromures de tétraalkylammonium, d'alkylamidoalkyl trialkylammonium, de trialkylbenzylammonium, de trialkylhydroxyalkylammonium, de dialkylamidoalkyldiméthylammonium, d'alkylpyridinium et les dérivés d'imidazolium.

Le véhicule aqueux des compositions selon l'invention est soit de l'eau soit un mélange d'eau et d'un solvant cosmétiquement acceptable tel que l'éthanol, le glycol ou un éther de glycol.

Les compositions selon l'invention peuvent également contenir au moins un additif cosmétiquement ou dermatologiquement acceptable choisi parmi un agent conditionneur, un agent épaississant, un polymère de type cationique, anionique, non-ionique ou amphotère, un filtre solaire, une céramide, un α-hydroxyacide, un agent conservateur, un agent anti-microbien, un agent anti-pelliculaire additionnel, un agent nacrant, un agent colorant, un parfum, un électrolyte ou un agent de mise en suspension.

Comme agent conditionneur pouvant être utilisé dans les compositions selon l'invention, on peut citer notamment les huiles naturelles hydrogénées ou non, les huiles synthétiques hydrocarbonées, cycliques ou aliphatiques, linéaires ou ramifiées, saturées ou insaturées, telles que par exemple les poly α-oléfines, en particulier les polydécènes et polyisobutènes, les huiles de silicone volatiles ou non, organo-modifiées ou non, solubles ou non, les huiles fluorées ou perfluorées, les esters gras, les esters d'alcools polyhydriques et les glycérides.

On peut également utiliser comme agent conditionneur dans les compositions selon l'invention, des cires synthétiques ou naturelles, des gommes et résines de silicone, des protéines ou des hydrolysats de protéine quaternisés ou non ou un mélange de ces divers agents.

Les additifs sont généralement présents dans les compositions selon l'invention en une proportion comprise entre 0,01 % et 20 % en poids, et de préférence entre 0,02 % et 10 % en poids par rapport au poids total de la composition.

Le pH des compositions selon l'invention est généralement inférieur à 7 et de préférence compris entre 3 et 4,5.

La présente invention a également pour objet un procédé de traitement cosmétique comprenant l'application sur la chevelure d'une quantité suffisante d'une composition telle que définie précédemment.

En général, la composition est appliquée 1 à 3 fois par semaine durant 6 à 8 semaines.

Lorsque l'on applique la composition selon l'invention sous forme d'une lotion ou d'une crème avant ou après shampooing, on la laisse éventuellement pauser sur la chevelure pendant environ 1/2 min à 5 min, puis on rince éventuellement à l'eau.

On va maintenant donner à titre d'illustration, plusieurs exemples de compositions capillaires anti-pelliculaires selon l'invention.

### EXEMPLE I : Shampooing anti-pelliculaire

- Disulfure de sélénium 0,25 g
- Chlorure de zinc 0,1 g
- Lauryléthersulfate de sodium et de magnésium (80/20) oxyéthyléné à 4 moles d'oxyde d'éthylène commercialisé sous la dénomination de "Empicol BSD"® par la Société Albright et Wilson 10 g MA
- Mélange cocoylamidopropylbétaïne/monolaurate de glycérol commercialisé sous la dénomination de "Tégobétaïne-HS"® par la Société Goldsmidt en solution aqueuse à 30 % de MA 5 g MA
- Polydiméthylsiloxane (PM 250.000) commercialisé sous la dénomination de "Silbione huile 70047 V 500.000"® par la Société Rhône Poulenc 2 g
- Mélange d'éther de cétyle et d'hydroxy-2 cétylstéaryl/alcool cétylique (60/40) 2,5 g
- Mono-isopropanolamide d'acide de coprah 1,5 g
- Copolymère acrylate d'alkyle (C₁₀₋C₃₀)-acide acrylique réticulé commercialisé sous la dénomination de "Carbopol 1382"® par la Société Goodrich 0,5 g
- NaOH q.s. pH 4
- Eau q.s.p. 100 g

Le shampooing ainsi obtenu, placé dans une étuve à 45°C pendant 1 mois, ne présente pas de modification de couleur et conserve son activité anti-pelliculaire.

Le même shampooing que ci-dessus, mais en l'absence du chlorure de zinc, change rapidement de couleur passant d'un orangé clair à un brun foncé.

Après avoir humidifié les cheveux, on applique une quantité suffisante du shampooing puis on fait mousser et laisse pauser environ 2 min. On rince ensuite abondamment la chevelure. Ce shampooing utilisé régulièrement permet d'éliminer et de prévenir la réapparition des pellicules.

### EXEMPLE II : I

- Disulfure de sélénium 1 g
- Chlorure de zinc 0,4 g
- Dodécanediol polyglycérolé à 3,5 moles de glycérol 20 g MA
- Perhydrosqualène 2 g
- Gomme de xanthane 1 g
- Conservateur q.s.
- Parfum q.s.
- Acide chlorhydrique q.s. pH 4
- Eau q.s.p. 100 g

### EXEMPLE III : Shampooing anti-pelliculaire

- Disulfure de sélénium 1 g
- Lactate de zinc 0,1 g
- Lauryléthersulfate de sodium et de magnésium (80/20) oxyéthyléné (Empicol BSD®) 10 g
- Mélange cocoylamidopropylbétaïne/monolaurate de glycérol (Tégobétaïne-HS®) 5 g MA
- Polydiméthylsiloxane (Silbione huile 70047 C 500.000®) 2 g
- Copolymère acrylate d'alkyle (C₁₀₋C₃₀)-acide acrylique réticulé (Carbopol 1382®) 0,5 g
- Mélange d'éther de cétyle et d'hydroxy-2 cétylstéaryl/ alcool cétylique (60/40) 2,5 g
- Mono-isopropanolamide d'acide de coprah 1,5 g
- NaOH q.s. pH 4
- Eau q.s.p. 100 g

### EXEMPLE IV : Composition anti-pelliculaire après-shampooing

- Disulfure de sélénium 1 g
- Chlorure de zinc 1 g
- Polyacrylamide commercialisé sous la dénomination de "Sepigel 305"® par la Société Seppic 3 g MA
- Cyclométhicone (et) diméthiconol commercialisé sous la dénomination de "Dow Corning 1401 Substantivity Aid Fluid®" par la Société Dow Corning 20 g
- Acide chlorhydrique q.s. pH 4
- Eau q.s.p. 100 g

### EXEMPLE V : Composition anti-pelliculaire après-shampooing

- Disulfure de sélénium 0,5 g
- Citrate de zinc 0,3 g
- Polyacrylamide ("Sepigel 305"®) 3 g MA
- Diphényl diméthicone commercialisée sous la dénomination de "Silbione Oil 70641 V 200"® par la Société Rhône-Poulenc 5 g
- Conservateur q.s.
- Parfum q.s.
- Acide chlorhydrique q.s. pH 4
- Eau q.s.p. 100 g

Après avoir abondamment rincé les cheveux préalablement soumis à un shampooing, on applique sur l'ensemble de la chevelure, une quantité suffisante de l'une des compositions des exemples IV et V. On laisse pauser pendant environ 2 à 5 min puis après avoir éventuellement rincé, on procède à la mise en forme de la chevelure.

## Revendications

1. Composition cosmétique, dermatologique ou pharmaceutique, stable, contenant en milieu aqueux du disulfure de sélénium, caractérisée par le fait que le disulfure de sélénium est présent en mélange avec au moins un sel de zinc d'un acide minéral ou organo-carboxylique.

2. Composition selon la revendication 1, caractérisée par le fait que ledit sel de zinc d'un acide minéral est choisi parmi le chlorure et le sulfate de zinc.

3. Composition selon la revendication 1, caractérisée par le fait que ledit sel de zinc d'un acide organo-carboxylique est choisi parmi l'acétate, le glycolate, le lactate, le gluconate et le citrate de zinc.

4. Composition selon l'une quelconque des revendications précédentes, caractérisée par le fait que le disulfure de sélénium est présent en une proportion comprise entre 0,001 % et 5 % en poids par rapport au poids total de la composition.

5. Composition selon la revendication 4, caractérisée par le fait que la proportion en disulfure de sélénium est comprise entre 0,25 % et 2 % en poids par rapport au poids total de la composition.

6. Composition selon l'une quelconque des revendications précédentes, caractérisée par le fait que ledit sel de zinc est présent en une proportion comprise entre 0,001 % et 5 % en poids par rapport au poids total de la composition.

7. Composition selon la revendication 6, caractérisée par le fait que la proportion en sel de zinc est comprise entre 0,1 % et 3 % en poids par rapport au poids total de la composition.

8. Composition selon l'une quelconque des revendications précédentes, caractérisée par le fait que le rapport en poids entre le sel de zinc et le disulfure de sélénium est compris entre 0,1 et 3.

9. Composition selon la revendication 8, caractérisée par le fait que ledit rapport est compris entre 0,2 et 2.

10. Composition selon l'une quelconque des revendications précédentes, caractérisée par le fait qu'elle contient en outre au moins un agent tensio-actif anionique, non-ionique, zwittérionique, amphotère ou cationique en une proportion comprise entre 0,01 % et 50 % en poids par rapport au poids total de la composition.

11. Composition selon l'une quelconque des revendications précédentes, caractérisée par le fait qu'elle contient en outre au moins un additif choisi parmi un agent conditionneur, un agent épaississant, un polymère du type cationique, anionique, non-ionique ou amphotère, un filtre solaire, une céramide, un α-hydroxyacide, un conservateur, un agent anti-microbien, un agent anti-pelliculaire additionnel, un agent nacrant, un agent colorant, un parfum, un électrolyte ou un agent de mise en suspension.

12. Procédé de traitement cosmétique, caractérisé par le fait que l'on applique sur la chevelure une quantité suffisante d'une composition selon l'une quelconque des revendications 1 à 11.

13. Utilisation d'au moins un sel de zinc d'un acide minéral ou organo-carboxylique pour la préparation d'une composition cosmétique, dermatologique ou pharmaceutique stable contenant en milieu aqueux du disulfure de sélénium, ladite composition étant destinée au traitement des pellicules.

14. Utilisation d'au moins un sel de zinc d'un acide minéral ou organo-carboxylique comme agent stabilisant d'une composition aqueuse contenant du disulfure de sélénium.

## Patentansprüche

1. Stabile kosmetische, dermatologische oder pharmazeutische Zubereitung, enthaltend in wäßrigem Milieu Selendisulfid, dadurch gekennzeichnet, dass das Selendisulfid im Gemisch mit mindestens einem Zinksalz einer Mineral- oder organischen Carbonsäure vorliegt.

2. Zubereitung nach Anspruch 1, dadurch gekennzeichnet, dass das Zinksalz einer Mineralsäure unter Zinkchlorid und Zinksulfat ausgewählt ist.

3. Zubereitung nach Anspruch 1, dadurch gekennzeichnet, dass das Zinksalz einer organischen Carbonsäure unter Zinkacetat, Zinkglycolat, Zinklactat, Zinkgluconat und Zinkcitrat ausgewählt ist.

4. Zubereitung nach einem der vorstehenden Ansprüche, dadurch gekennzeichnet, dass das Selendisulfid in einem Anteil zwischen 0,001 und 5 Gew.-%, bezogen auf das Gesamtgewicht der Zubereitung, vorliegt.

5. Zubereitung nach Anspruch 4, dadurch gekennzeichnet, dass der Anteil des Selendisulfids zwischen 0,25 und 2 Gew.-%, bezogen auf das Gesamtgewicht der Zubereitung.

6. Zubereitung nach einem der vorstehenden Ansprüche, dadurch gekennzeichnet, dass das Zinksalz in einem Anteil zwischen 0,001 und 5 Gew.-%, bezogen auf das Gesamtgewicht der Zubereitung, vorliegt.

7. Zubereitung nach Anspruch 6, dadurch gekennzeichnet, dass der Anteil des Zinksalzes zwischen 0,1 und 3 Gew.-%, bezogen auf das Gesamtgewicht der Zubereitung, vorliegt.

8. Zubereitung nach einem der vorstehenden Ansprüche, dadurch gekennzeichnet, dass das Gewichtsverhältnis zwischen dem Zinksalz und Selendisulfid zwischen 0,1 und 3 beträgt.

9. Zubereitung nach Anspruch 8, dadurch gekennzeichnet, dass das Verhältnis zwischen 0,2 und 2 beträgt.

10. Zubereitung nach einem der vorstehenden Ansprüche, dadurch gekennzeichnet, dass sie ausserdem mindestens ein anionisches, nicht-ionisches, zwitter-ionisches, amphoteres oder kationisches grenzflächenaktives Mittel in einem Anteil zwischen 0,01 und 50 Gew.-%, bezogen auf das Gesamtgewicht der Zubereitung, enthält.

11. Zubereitung nach einem der vorstehenden Ansprüche, dadurch gekennzeichnet, dass sie ausserdem mindestens ein Additiv enthält, ausgewählt unter Konditionierungsmittel, ein Verdickungsmittel, ein Polymer von kationischen, anionischen, nichtionischen oder amphoteren Typ, ein Sonnenfilter, ein Ceramid, eine α-Hydroxysäure, ein Konservierungsmittel, ein antimikrobielles Mittel, ein zusätzliches Mittel gegen Filmbildung, ein Mittel gegen Schuppenbildung, ein Färbemittel, ein Parfum, einen Elektrolyt oder ein Suspensionshilfsmittel enthält.

12. Kosmetisches Behandlungsverfahren, dadurch gekennzeichnet, daß man auf das Haar ausreichende Menge einer Zubereitung gemäß den Ansprüchen 1 bis 11 aufbringt.

13. Verwendung mindestens eines Zinksalzes einer Mineralsäure oder einer organischen Carbonsäure für die Herstellung eines stabilen kosmetischen, dermatologischen oder pharmazeutischen Zubereitung, enthaltend in wässrigem Milieu Selendisulfid, wobei die Zubereitung für die Schuppenbehandlung der Haare bestimmt ist.

14. Verwendung mindestens eines Zinksalzes einer Mineralsäure oder einer organischen Carbonsäure als Stabilisierungsmittel einer wässrigen Zubereitung, enthaltend Selendisulfid.

## Claims

1. Stable cosmetic, dermatological or pharmaceutical composition comprising selenium disulphide in aqueous medium, characterized in that the selenium disulphide is present as a mixture with at least one zinc salt of an inorganic or organocarboxylic acid.

2. Composition according to Claim 1, characterized in that the said zinc salt of an inorganic acid is chosen from zinc chloride and sulphate.

3. Composition according to Claim 1, characterized in that the said zinc salt of an organocarboxylic acid is chosen from zinc acetate, glycolate, lactate, gluconate and citrate.

4. Composition according to any one of the preceding claims, characterized in that the selenium disulphide is present in a proportion of between 0.001% and 5% by weight with respect to the total weight of the composition.

5. Composition according to Claim 4, characterized in that the proportion of selenium disulphide is between 0.25% and 2% by weight with respect to the total weight of the composition.

6. Composition according to any one of the preceding claims, characterized in that the said zinc salt is present in a proportion of between 0.001% and 5% by weight with respect to the total weight of the composition.

7. Composition according to Claim 6, characterized in that the proportion of zinc salt is between 0.1% and 3% by weight with respect to the total weight of the composition.

8. Composition according to any one of the preceding claims, characterized in that the ratio by weight of the zinc salt to the selenium disulphide is between 0.1 and 3.

9. Composition according to Claim 8, characterized in that the said ratio is between 0.2 and 2.

10. Composition according to any one of the preceding claims, characterized in that it additionally comprises at least one anionic, nonionic, zwitterionic, amphoteric or cationic surface-active agent in a proportion of between 0.01% and 50% by weight with respect to the total weight of the composition.

11. Composition according to any one of the preceding claims, characterized in that it additionally comprises at least one additive chosen from a conditioning agent, a thickening agent, a polymer of the cationic, anionic, nonionic or amphoteric type, a sunscreen agent, a ceramide, an α-hydroxy acid, a preservative, an antimicrobial agent, an additional antidandruff agent, a pearlescent agent, a colouring agent, a fragrance, an electrolyte or a suspending agent.

12. Cosmetic treatment process, characterized in that a sufficient amount of a composition according to any one of Claims 1 to 11 is applied to the hair.

13. Use of at least one zinc salt of an inorganic or organocarboxylic acid in the preparation of a stable cosmetic, dermatological or pharmaceutical composition comprising selenium disulphide in aqueous medium, the said composition being intended for the treatment of dandruff.

14. Use of at least one zinc salt of an inorganic or organocarboxylic acid as stabilizing agent for an aqueous composition comprising selenium disulphide.
